# EUROPEAN PATENT APPLICATION

(11) **EP 2 574 932 A1**
(43) Date of publication of application: **03.04.2013**
(21) Application number: 11183595.5
(22) Date of filing: 30.09.2011
(51) Int. Cl.: G01N 33/68

(54) **sFlt1 in subjects during or immediately after physical exercise**

(71) Applicant: Roche Diagnostics GmbH, 68305 Mannheim (DE); F. Hoffmann-La Roche AG, 4070 Basel (CH)
(72) Inventor: Hess, Georg, 55130 Mainz (DE); Horsch, Andrea, 68259 Mannheim (DE); Zdunek, Dietmar, 82327 Tutzing (DE)
(74) Representative: Dick, Alexander

(57) **Abstract**

The present invention relates to a method for diagnosing in a subject who participates in exercise and who exhibits chest pain whether chest pain is associated with cardiac ischemia, or not. The method is based on the determination of the amount of soluble fms-like tyrosine kinase-1 (sFlt1) in a sample from said subject obtained during or immediately after physical exercise, and on the comparison of the thus determined amount of sFlt1 to a reference amount. In the context of the method of the present invention, the reference amount shall be derived from a sample from a reference subject obtained during or immediately after physical exercise, or from a group thereof.

## Description

The present invention relates to a method for diagnosing in a subject who participates in exercise and who exhibits chest pain whether the chest pain is associated with cardiac ischemia, or not. The method is based on the determination of the amount of soluble fms-like tyrosine kinase-1 (sFlt1) in a sample from said subject obtained during or immediately after physical exercise, and on the comparison of the thus determined amount of sFlt1 to a reference amount. In the context of the method of the present invention, the reference amount shall be derived from a sample from a reference subject obtained during or immediately after physical exercise, or from a group thereof.

Chest pain is a frequent cause for visit in the emergency room, this occurs in approximately 7 million persons in the USA each year. Approximately 15 to 25 percent of these individuals are diagnosed with acute coronary syndrome. One difficulty is to discriminate cardiac from non-cardiac chest pain (Cannon C.P. and Lee T.H., chapter 49 p 1195 ff in Braunwalds Heart Disease). Cardiac chest pain is caused by a mismatch between perfusion pressure and myocardial oxygen demand which is the case in aortic stenosis or hypertrophic cardiomyopathy. Additional causes include increased heart rate, anemia etc. One specific precipitating cause of chest pain and ACS is exercise, during exercise heart rate increases and the oxygen demand does too. In case of pre-existent coronary artery disease which might be known or unknown to the person chest pain might develop, that takes the person to the emergency room. With the development of highly sensitive assays for the detection of troponins and specifically troponin T, increases of troponin of 30 to 100 % have been considered to reflect non ST elevation myocardial infarction.

Recent publications indicated that physical exercise may increase the level of cardiac biomarkers. Scharhag et al. (Med Sci Sports Exerc. 2008 Aug;40(8): 1408-15) discloses that cardiac biomarkers, e.g. cardiac troponins, can be increased after physical exercise. According to Scharhag, it would be still unclear whether exercise-associated increases of cardiac biomarkers represent a clinically relevant insult or are part of the physiological response to endurance exercise. The levels may correspond to Troponin levels associated with ST elevations and, thus, mimick ST elevation myocardial infarction (STEMI).

Whyte (Med Sci Sports Exerc. 2008 Aug;40(8):1416-23) discloses that cardiac troponins are increased after endurance exercise in elite and recreational athletes. Whyte further discloses that endurance exercise may act as a promoter for right ventricular remodeling and a resultant trigger for ventricular arrhythmia.

Soluble fms-like tyrosine kinase-1 (sFlt-1 or sVEGFR-1) is a tyrosine kinase protein that disables proteins that cause blood vessel growth. Soluble Flt-1 (sFlt-1) is a splice variant of VEGF receptor 1 (Flt-1) which is produced by a variety of tissues. US2007218498 (Büchler) discloses a method diagnosing or predicting the risk to suffer from a cardiovascular complication including ACS, NSTEMI and unstable angina based on the detection of sFlt1 (Soluble fms-like tyrosine kinase-1). Also disclosed is a method of monitoring the treatment for a patient diagnosed with a cardiovascular disease, e.g. ACS. However, Büchler does not disclose the determination of sFlt1 in subjects participating in exercise.

WO2010/075475 discloses a method of determining the risk of a patient presenting with acute chest pain to suffer from a major acute cardiovascular event (MACE) within one year following presentation based on the detection of sFlt1. WO2010/075475 does not disclose the determination of sFlt1 in subjects participating in physical exercise.

sFlt1 has been proposed as a marker of cardiac ischemia (see W02010/075475). Interestingly however, it has been found in the studies carried out in the context of the present invention, that sFlt1 levels are increased during and immediately after physical exercise. Depending on exercise intensity, the sFlt1 levels observed during or immediately after physical exercise correspond to sFlt1 levels observed in patients with ACS.

It has been found that increased sFlt1 levels in subjects during or after physical exercise are usually not a sign of pathological cardiac ischemia, i.e. of cardiac ischemia which requires intervention. Rather, only significantly increased levels of sFlt1 during or immediately after physical exercise are indicative for pathological cardiac ischemia.

In subjects exhibiting chest pain during or immediately after physical exercise, the biomarker sFlt1 can be used for diagnosing whether chest pain has been caused by cardiac ischemia. However, the reference amount for carrying out the diagnosis has to be derived from a subject during or immediately after physical exercise, rather than from a subject at rest. The use of reference amounts derived from a subject at rest would lead to a large number of false positive results. In contrast, when using a reference amount derived from a subject during or immediately after physical exercise, it can be reliably ruled out that chest pain in a subject participating in exercise is not associated with cardiac ischemia.

Thus, conventional diagnostic techniques based on sFlt1 usually do not allow for a reliable diagnosis of subjects during or immediately after physical exercise. Accordingly, a personalized treatment regimen can not be determined with sufficient accuracy. As a consequence thereof, many patients will receive a treatment regimen which is insufficient or which may have adverse side effects. In many cases, acute cardiovascular events, once determined by the conventional diagnostic techniques referred to above are currently treated by cardiac interventions. Those cardiac interventions include various types of angioplasty- based interventions and/or coronary bypass surgery which are carried out in order to restore proper blood flow, e.g., within the coronary vessels. However, those interventions may be even harmful for the patient. In addition, the interventions are time and cost expensive. Therefore, means and methods are required for reliably diagnosing whether chest pain in subjects participating in exercise is associated with cardiac ischemia.

In the prior art (e.g. Sharhag et al., Whyte et al.), it was shown that cardiac Troponins can be increased after physical exercise. This was confirmed in the studies carried out by the inventors. However, it has been surprisingly observed that the increase of sFlt1 can be observed already early, i.e. during or immediately after physical exercise. Thus, the level of sFlt1 provides earlier diagnostic information than a cardiac Troponin. Accordingly, early diagnostic information may -based on the level of sFlt1- particularly allow an early rule out of cardiac ischemia in a subject who participates in exercise and who presents with chest pain. This will allow for relieving anxiety in said subject.

There is a need for diagnostic which allow for a reliable diagnosis in a subject exhibiting chest pain during or immediately after physical exercise is associated cardiac ischemia.

The technical problem underlying the present invention can be seen as the provision of means and methods for complying with the aforementioned need.

The technical problem is solved by the embodiments characterized in the claims and herein below.

Accordingly, the present invention relates to method for diagnosing whether chest pain is associated with cardiac ischemia in a subject during or immediately after physical exercise, comprising the step of determining the amount of soluble fms-like tyrosine kinase-1 (sFlt1) in a sample obtained during or immediately after physical exercise from a subject exhibiting chest pain, wherein the reference amount is derived from a sample from a reference subject obtained during or immediately after physical exercise, or from a group thereof.

In a preferred embodiment the method comprises the further step of b) comparing the, thus, determined amount of sFlt1 to a reference amount.

Preferably, it is diagnosed thereby, whether chest pain is associated with cardiac ischemia.

Accordingly, the present invention, in particular, relates to method for diagnosing whether chest pain is associated with cardiac ischemia in a subject during or immediately after physical exercise, comprising
a) determining the amount of soluble fms-like tyrosine kinase-1 (sFlt1) in a sample obtained during or immediately after physical exercise from a subject exhibiting chest pain, and
b) comparing the, thus, determined amount of sFlt1 to a reference amount, whereby it is diagnosed whether chest pain is associated with cardiac ischemia,
   wherein the reference amount is derived from a sample from a reference subject obtained during or immediately after physical exercise, or from a group thereof.

Preferably, it is diagnosed, whether chest pain is associated with cardiac ischemia by carrying out the further step of c) diagnosing whether chest pain is associated with cardiac ischemia, or not, based on the result of the comparison carried out in step b).

The method of the present invention, preferably, is an ex vivo method. Moreover, it may comprise steps in addition to those explicitly mentioned above. For example, further steps may relate to sample pre-treatments or evaluation of the results obtained by the method. The method may be carried out manually or assisted by automation. Preferably, step (a) and/or (b) may in total or in part be assisted by automation, e.g., by a suitable robotic and sensory equipment for the determination in step (a) or a computer-implemented comparison and/or diagnosis based on said comparison in step (b).

The term "diagnosing" as used herein means assessing whether chest pain is associated with cardiac ischemia in a subj ect as referred to in accordance with the method of the present invention, or not. As will be understood by those skilled in the art, such an assessment is usually not intended to be correct for 100% of the subjects to be diagnosed. The term, however, requires that the assessment is correct for a statistically significant portion of the subjects (e.g. a cohort in a cohort study). Thus, the method of the present invention, however, at least provides an aid for establishing a final clinical diagnosis. Whether a portion is statistically significant, can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

The term "cardiac ischemia" is well known in the art. As used herein, the term, preferably, refers to a condition characterized by a reduced blood flow to the heart leading to a reduced delivery of oxygen delivery (undersupply) to cells comprised by the cardiac tissue.

Preferably, cardiac tissue is affected by ischemia, if the amount of oxygen that is supplied to said tissue is not sufficient in order to cover the need of the cells comprised by said tissue, and, thus, to meet the rate of mitochondrial oxidation in said cells. As a consequence of said reduced delivery myocardial stunning or hibernation and even myocardial necrosis may occur. In particular, the term "cardiac ischemia" relates to myocardial ischemia.

In the context of the present invention is shall be assessed whether chest pain is associated, in particular caused by, cardiac ischemia. The term "chest pain" includes any pain that occurs in the area between the neck and the bottom of the rib cage. The chest pain may have several causes. In principle, any part of the chest can be the cause of the pain including the heart, lungs, esophagus, muscle, bone, and skin. The terms "chest pain" as used herein is generally known to the skilled physician. In particular, the chest pain may be caused cardiac ischemia in particular by an acute coronary event. Chest pain may also have other causes than cardiac ischemia, e.g. by heart failure. Preferred non-cardiac causes of chest pain include musculosceletal chest pain, pleuritis, pneumonia, pneumothorax, gastritis, gastro esophageal reflux disease, and anxiety disorder. Preferably, the chest pain has a duration of at least 5, in particular of at least 20 minutes. The chest pain may persist or may not persist at the time the sample to be tested is obtained. Is it particularly envisaged that the onset of chest pain was after the start of physical exercise.

The term "associated with" as used herein, preferably, refers to a temporal and/or causal relationship between cardiac ischemia and chest pain. The person skilled in the art understands what is meant if chest pain is considered to be associated with cardiac ischemia. Preferably, chest pain shall be considered to be associated cardiac ischemia if the chest pain is caused by said cardiac ischemia, whether directly or indirectly. Thus, with the expression "chest pain associated with cardiac ischemia" it is, preferably, meant that cardiac ischemia is the cause of chest pain. Indication for such causal connection is in particular a close time-relationship between cardiac ischemia and chest pain.

The term "subject" as used herein relates to animals, preferably mammals, and, more preferably, humans. The subject can be male or female. It is however preferred that the subject is male. The subject according to the present invention shall, preferably, suffer from chest pain as described elsewhere herein. Preferably, the subject does not exhibit impaired renal function.

The subject to be tested, preferably, participates in physical exercise, at the time at which the sample to be tested is obtained, or immediately before the sample to be tested is obtained. The term "exercise" as used herein, preferably, refers to any form physical activity. Preferably, the exercise enhances or maintains physical fitness. The term "exercise", preferably, refers to strength exercise, and, more preferably, endurance exercise. The terms "endurance exercise" and "strength exercise" are well known in the art. Whereas endurance exercise, in particular, aims to increase cardiovascular endurance, strength exercise aims to increase short-term muscle strength. Preferred endurance exercises include cycling, swimming, rope, rowing, running, playing tennis and triathlon. Particularly preferred endurance exercises are running and cycling and swimming. Preferred strength exercises include weight training, eccentric training and sprinting.

As described elsewhere herein, the sample to be tested shall be either obtained during physical exercise or after physical exercise. It the sample is obtained after physical exerciser, the subject, preferably, participated in physical exercise for at least 30 minutes. More preferably, the subject participated in physical exercise for at least 60 minutes. More preferably, the subject participated in physical exercise for at least 90 minutes. Also envisaged by the present invention is a physical exercise of at least 10 min. Preferably, however, the duration of physical exercise does not exceed three hours. More preferably, the duration of physical exercise does not exceed two hours. Accordingly, the subject preferably participated in exercise for a period of 10 to 120 or 180 minutes.

Preferably, the exercise intensity is at least 70% of the anaerobic threshold, more preferably, at least 90%, or most preferably at least 110% of the anaerobic threshold. As will be understood by the skilled person, the exercise intensity may vary during exercise. However, it is contemplated that the said exercise intensities are reached at least once during physical exercise, preferably, for at least 5 minutes, more preferably, for at least 10 or 15 minutes, or even more preferably, for at least 15 minutes, and most preferably, for at least 30 minutes.

The term "anaerobic threshold" is well known in the art. Preferably, the anaerobic threshold is the point above which the muscles derive their energy from nonoxygenic sources rather than oxygenic sources during exercise. It is well known in the art that above the anaerobic threshold lactate (i.e. lactic acid) is produced faster than it is metabolized in the body. Accordingly, lactate accumulates in the blood when the exercise intensity is above the anaerobic threshold. When the exercise intensity is below the anaerobic threshold, lactate does not accumulate in the body. The anaerobic threshold can be determined by well known methods, e.g. by measuring lactate threshold involves in blood samples obtained during a ramp test where the exercise intensity is progressively increased. The anaerobic threshold is reached when lactic acid starts to accumulate. The threshold can also be performed non-invasively using gas-exchange methods in which the composition of the air inspired and expired is determined. Preferably, the anaerobic threshold is determined by a stepwise exercise test on a cycle ergometer using the method of Stegmann H. Stegmann, W. Kindermann, A. Schnabel, Lactate Kinetics and Individual Anaerobic Threshold, Int J Sports Med 1981; 02(3): 160-165, which is herewith incorporated by reference with respect to its entire disclosure content. The method is, preferably, based on determining the gas exchange and the maximal oxygen consumption.

The term "sample" refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma or serum. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce the peptides referred to herein.

Preferably, the sample is obtained during or after physical exercise from the subject exhibiting chest pain. It is particularly preferred that the sample is obtained immediately after physical exercise. Accordingly, the sample is, preferably, obtained not more than 120 minutes, more preferably, not more than 90 or 60 minutes, even more preferably, not more than 45 minutes and, most preferably, not more than 30 or 15 minutes after physical exercise, i.e. after the end of physical exercise (or, alternatively, after the maximum training intensity). It is further preferred to obtain the sample at the end of physical exercise.

The term "soluble Flt-1" or "sFlt-1" (abbreviation for Soluble fms-like tyrosine kinase-1) as used herein refers to polypeptide which is a soluble form of the VEGF receptor Flt1. It was identified in conditioned culture medium of human umbilical vein endothelial cells. The endogenous soluble Flt1 (sFlt1) receptor is chromatographically and immunologically similar to recombinant human sFlt1 and binds [125I] VEGF with a comparable high affinity. Human sFlt1 is shown to form a VEGF-stabilized complex with the extracellular domain of KDR/Flk-1 in vitro. Preferably, sFlt1 refers to human sFlt1 as described in Kendall 1996, Biochem Biophs Res Commun 226(2): 324-328 (for amino acid sequences, see, e.g., also P17948, GI: 125361 for human and BAA24499.1, GI: 2809071 for mouse sFlt-1). The term also encompasses variants of the aforementioned human sFlt-1 polypeptides. Such variants have at least the same essential biological and immunological properties as the aforementioned sFlt-1 polypeptide. In particular, they share the same essential biological and immunological properties if they are detectable by the same specific assays referred to in this specification, e.g., by ELISA assays using polyclonal or monoclonal antibodies specifically recognizing the said sFlt-1 polypeptides. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific sFlt-1 polypeptide, preferably over the entire length of the human sFlt-1, respectively. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Moreover, the variants referred to herein include fragments or subunits of the specific sFlt-1 polypeptides or the aforementioned types of variants as long as these fragments have the essential immunological and biological properties as referred to above. Such fragments may be, e.g., degradation products of the sFlt-1 polypeptides. Further included are variants which differ due to posttranslational modifications such as phosphorylation or myristylation.

Determining the amount of a peptide or polypeptide referred to in this specification relates to measuring the amount or concentration, preferably, semi-quantitatively or quantitatively. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay and methods which may utilize labeled molecules in various sandwich, competition, or other assay formats. Such assays are, preferably, based on detection agents such as antibodies which specifically recognize the peptide or polypeptide to be determined. The detection agents shall be either directly or indirectly capable of generating a signal indicating the presence or absence of the peptide or polypeptide. Moreover, the signal strength can, preferably, be correlated directly or indirectly (e.g. reverse- proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immuno-assays (available for example on Elecsys^{™} analyzers), CBA (an enzymatic Cobalt Binding Assay, available for example on Roche-Hitachi^{™} analyzers), and latex agglutination assays (available for example on Roche-Hitachi^{™} analyzers).

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide, preferably, comprises the steps of (a) contacting the peptide with a specific ligand, (b) (optionally) removing non-bound ligand, (c) measuring the amount of bound ligand. The bound ligand will generate an intensity signal. Binding according to the present invention includes both covalent and non-covalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semi-quantitative or quantitative. Further suitable techniques for the determination of a polypeptide or peptide are described in the following.

First, binding of a ligand may be measured directly, e.g. by NMR or surface plasmon resonance. Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labeled with a detectable lable prior to the reaction. Preferably, the sample is contacted with the substrate for an adequate period of time. An adequate period of time refers to the time necessary for an detectable, preferably measurable, amount of product to be produced. Instead of measuring the amount of product, the time necessary for appearance of a given (e.g. detectable) amount of product can be measured. Third, the ligand may be coupled covalently or non-covalently to a label allowing detection and measurement of the ligand. Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager. Suitable measurement methods according the present invention also include precipitation (particularly im-munoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labeling or other dectection methods as described above.

The amount of a peptide or polypeptide may be, also preferably, determined as follows: (a) contacting a solid support comprising a ligand for the peptide or polypeptide as specified above with a sample comprising the peptide or polypeptide and (b) measuring the amount peptide or polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Bio-technol. 20(1):9-12). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are generally known (US 5,744,305).

The term "amount" as used herein encompasses the absolute amount of a polypeptide or peptide, the relative amount or concentration of the said polypeptide or peptide as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned amounts or parameters can also be obtained by all standard mathematical operations.

It was shown in the context of the invention, that subjects who participate in exercise may show sFlt1 levels that correspond to the levels of sFlt1 in patients with unstable angina. However, none of the subjects analyzed suffered from unstable angina. Thus, the increased sFlt1-levels may be a sign of peripheral ischemia, i.e. of ischemia in tissues other than the myocardium. Thus, in a preferred embodiment of the method of the present invention, the amount of sFlt1 determined in step a) corresponds to the amount of sFlt1 in a sample from a subject who did not participate in physical exercise and who suffers from unstable angina, in particular to the amount of sFlt1 in a sample from a subject suffering from unstable angina occurring at rest (or minimal exertion). The term "unstable angina" is well known in the art. Unstable angina is usually defined as angina pectoris with at least one of the three features: 1. occurring at rest or minimal, exertion and usually lasting more than 20 minutes, 2. being severe and described as frank pain and of new onset und 3. occurring with a crescendo. Thus, the amount of sFlt1 determined in step a) corresponds to the amount of sFlt1 in a sample from a subject with unstable angina obtained at rest from said subject. Preferably, the subject with unstable angina did not participate in physical exercise within 2 hours prior to obtaining the sample.

The term "comparing" as used herein encompasses comparing the amount of the peptide or polypeptide comprised by the sample to be analyzed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provide the desired assessment in a suitable output format. Based on the comparison of the amount determined in step a) and the reference amount, it is possible to diagnose whether chest pain is associated with cardiac ischemia, or not.

The reference amount to be applied in the context of the method of the present invention, is preferably, derived from a sample from a reference subject obtained during or immediately after physical exercise, or from a group of reference subjects. The term "physical exercise" has been described elsewhere herein. Preferably, the intensity, duration and kind of exercise carried out by the reference subject correspond to intensity, duration and kind of exercise carried out by the subject to be tested. Moreover, the sample from the reference subject(s) is preferably obtained at the same time with respect to physical exercise.

The term "reference amount" as used herein refers to an amount which allows for allocation of a subject into either the group of subjects in which chest pain is associated with cardiac ischemia or into the group of subjects in which chest pain is not associated with cardiac ischemia. Such a reference amount can be a threshold amount which separates these groups from each other. Accordingly, the reference amount for a the biomarker sFlt1 shall be an amount which allows for allocation of a subject into a group of in which chest pain is associated with cardiac ischemia or into the group of subjects in which chest pain is not associated with cardiac ischemia. A suitable threshold amount separating the two groups can be calculated without further ado by the statistical tests referred to herein elsewhere based on amounts of a sFlt1 from either a subject or group of subjects known to exhibit chest pain associated with cardiac ischemia, or from or from a subject or group of subjects known to exhibit chest pain not associated with cardiac ischemia. during and/or immediately after physical exercise. Preferred referenced amounts which can be derived from the aforementioned subjects or group of subjects are indicated elsewhere herein.

Preferably, the reference subject is known to exhibit chest pain not associated with cardiac ischemia (in particular during or immediately after physical exercise). In this case, an amount of sFlt1 in the sample of the test subject which is the same, in particular essentially the same, or which is decreased as compared to the reference amount indicates indicates that the test subject exhibits chest pain not associated with cardiac ischemia.

Also preferably, the reference subject is known exhibit chest pain associated with cardiac ischemia (in particular during or immediately after physical exercise). In this case, an amount of sFlt1 in the sample of the test subject which is the same, in particular essentially the same, or which is increased as compared to the reference amount indicates that the test subject exhibits chest pain associated with cardiac ischemia.

Moreover, the reference amount may define a threshold amount, whereby an amount larger than the threshold shall be indicative for a subject exhibiting chest pain associated with cardiac ischemia while an amount lower than the threshold amount shall be an indicator for a subject exhibiting chest pain not associated with chest pain. The reference amount applicable for an individual subject may vary depending on various physiological parameters such as age, gender, or subpopulation, as well as on the means used for the determination of the polypeptide or peptide referred to herein. A suitable reference amount may be determined by the method of the present invention from a reference sample to be analyzed together, i.e., simultaneously or subsequently, with the test sample. A preferred reference amount serving as a threshold may be derived from the upper limit of normal (ULN), i.e., the upper limit of the physiological amount to be found in samples derived from a population of subjects during or immediately after physical exercise. The ULN for a given population of subjects can be determined by various well known techniques. A preferred threshold (i.e., reference amount which is preferably derived from a reference subject referred to herein) for sFlt is at least one to preferably, 200 pg/ml, more preferably, 250 pg/ml and most preferably, 300 pg/ml. An amount in the sample of a test subject lower than the reference amount, preferably, indicates that chest pain is not associated with cardiac ischemia, whereas An amount in the sample of a test subject larger than the reference amount indicates that chest pain is associated with cardiac ischemia.

In a preferred embodiment, the method of the present invention further comprises the step of recommending a cardiac therapy and/or a further diagnostic measure in case of the diagnosis that the chest pain is associated with cardiac ischemia.

The term "recommending" as used herein means establishing a proposal for a therapy which could be applied to the subject. However, it is to be understood that applying the actual therapy whatsoever is not comprised by the term. The therapy to be recommended depends on the outcome of the diagnosis provided by the method of the present invention. Preferably, the cardiac therapy aims to ameliorate, i.e. reduce, cardiac ischemia in said subject. A preferred cardiac therapy is selected from the group consisting of administration of nitroglycerin (1,2,3-trinitroxypropane), oxygen, clopridogel and heparin.

A preferred further diagnostic measure is coronary angiography.

In a further preferred embodiment of the method of the present invention further comprises the determination of GDF-15 in the sample from the subject, and comparing, the, thus, determined amount to a reference amount.

GDF-15 is a well known marker for heart failure Kempf et al. (Circulation Research 2006: 98: 351 - 360). Preferably, GDF-15 is determined in sample from subjects exhibiting chest pain associated with cardiac ischemia. Preferably, the determination of GDF-15 allows for recommending percutaneous coronary intervention (PCI). Preferably, an amount of GDF-15 which is increased with respect to the reference amount is indicative that the PCI will be beneficial, wherein an amount of GDF-15 lower than the reference amount indicates that PCI would not be beneficial (and thus, would not be required and would put the subject at risk.

The term "growth differentiation factor-15" or "GDF-15" relates to polypeptide being a member of the transforming growth factor (TGF)-beta cytokine superfamily. The terms polypeptide, peptide, and protein are used interchangeably throughout this specification. GDF-15 was originally cloned as macrophage-inhibitory cytokine-1 and later also identified as placental transforming growth factor-.beta., placental bone morphogenetic protein, non-steroidal anti-inflammatory drug-activated gene-1, and prostate-derived factor (Bootcov loc cit; Hromas, 1997 Biochim Biophys Acta 1354:40-44; Lawton 1997, Gene 203:17-26; Yokoyama-Kobayashi 1997, J Biochem (Tokyo), 122:622-626; Paralkar 1998, J Biol Chem 273:13760-13767). Similar to other TGF-.beta.-related cytokines, GDF-15 is synthesized as an inactive precursor protein, which undergoes disulfide-linked homodimerization. Upon proteolytic cleavage of the N-terminal pro-peptide, GDF-15 is secreted as a about.28 kDa; dimeric protein (Bauskin 2000, Embo J 19:2212-2220). Amino acid sequences for GDF-15 are disclosed in WO99/06445, WO00/70051, WO2005/113585, Bott-ner 1999, Gene 237: 105-111, Bootcov loc. cit, Tan loc. cit., Baek 2001, Mol Pharmacol 59: 901-908, Hromas loc cit, Paralkar loc cit, Morrish 1996, Placenta 17:431-441 or Yokoyama-Kobayashi loc cit. GDF-15 as used herein encompasses, also variants of the aforementioned specific GDF-15 polypeptides. Such variants have at least the same essential biological and immunological properties as the specific GDF-15 polypeptides. In particular, they share the same essential biological and immunological properties if they are detectable by the same specific assays referred to in this specification, e.g., by ELISA assays using polyclonal or monoclonal antibodies specifically recognizing the said GDF-15 polypeptides. A preferred assay is described in the accompanying Examples. Moreover, it is to be understood that a variant as referred to in accordance with the present invention shall have an amino acid sequence which differs due to at least the one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific GDF-15 polypeptides (in particular over the entire length). The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, Wis.), or by visual inspection. Given that two sequences have been identified for comparison, GAP and BESTFIT are preferably employed to determine their optimal alignment and, thus, the degree of identify. Preferably, the default values of 5.00 for gap weight and 0.30 for gap weight length are used. Variants referred to above may be allelic variants or any other species specific homologs, paralogs, or orthologs. Moreover, the variants referred to herein include fragments of the specific GDF-15 polypeptides or the aforementioned types of variants as long as these fragments have the essential immunological and biological properties as referred to above. Such fragments may be, e.g., degradation products of the GDF-15 polypeptides. Further included are variants which differ due to posttranslational modifications such as phosphorylation or myristylation.

The present invention also envisages the use of sFlt1, or of a detection agent, which specifically binds thereto, in a sample obtained during or immediately after physical exercise from a subject exhibiting chest pain, for assessing whether the chest pain is associated with cardiac ischemia.

The present invention also envisages the use of sFlt1 and GDF15, or of a detection agents, which specifically binds thereto, in a sample obtained during or immediately after physical exercise from a subject exhibiting chest pain, for assessing whether the chest pain is associated with cardiac ischemia.

The term "detection agent" as used herein refers to an agent which is capable of specifically recognizing and binding the biomarker referred to herein (sFlt1 or GDF-15) when present in a sample. Moreover, said agent shall allow for direct or indirect detection of the complex formed by the said agent and the biomarker. Direct detection can be achieved by including into the agent a detectable label. Indirect labelling may be achieved by a further agent which specifically binds to the complex comprising the biomarker and the detection agent wherein the said further agent is than capable of generating a detectable signal. Suitable compounds which can be used as detection agents are well known in the art. Preferably, the detection agent is an antibody or aptamere which specifically binds to the biomarker. Antibodies as referred to herein include both polyclonal and monoclonal antibodies, as well as fragments thereof, such as Fv, Fab and F(ab)₂ fragments that are capable of binding antigen or hapten. Also envisaged are single chain antibodies and humanized hybrid antibodies wherein amino acid sequences of a non-human donor antibody exhibiting a desired antigen-specificity are combined with sequences of a human acceptor antibody.

The present invention further relates to a device for diagnosing whether chest pain is associated with cardiac ischemia in a subject during or immediately after physical exercise, or not, said device comprising:
a) an analyzing unit comprising a detection agent for sFlt1 which allows for the determination of the amount of sFlt1; and
b) an evaluation unit comprising a data processor having implemented an algorithm for comparing the amounts determined by the analyzing unit with reference amounts stored in a database in order to establish a diagnosis indicating whether chest pain is associated with cardiac ischemia in a subject during or immediately after physical exercise, or not, wherein the reference amount is derived from a sample from a reference subject obtained during or immediately after physical exercise or group thereof, and the algorithm is an algorithm as defined above in connection with the method of the invention.

The term "device" as used herein relates to a system comprising the aforementioned units operatively linked to each other as to allow the diagnosis according to the methods of the invention. Preferred detection agents which can be used for the analyzing unit are disclosed elsewhere herein. The analyzing unit, preferably, comprises said detection agents in immobilized form on a solid support which is to be contacted to the sample comprising the biomarkers the amount of which is to be determined. Moreover, the analyzing unit can also comprise a detector which determines the amount of detection agent which is specifically bound to the biomarker(s). The determined amount can be transmitted to the evaluation unit. Said evaluation unit comprises a data processing element, such as a computer, with an implemented algorithm for carrying out a comparison between the determined amount and a suitable reference. Suitable references can be derived from samples of subjects to be used for the generation of reference amounts as described elsewhere herein above. The diagnostic results may be given as output of parametric diagnostic raw data, preferably, as absolute or relative amounts. It is to be understood that these data may need interpretation by the clinician. However, also envisage are expert system devices wherein the output comprises processed diagnostic raw data the interpretation of which does not require a specialized clinician. Preferably, the device of the present invention can be used to carry out the aforementioned method of the present invention in an automated manner.

In a preferred embodiment of the device of the present invention, said evaluation unit further comprises a database with recommendations for a cardiac therapy and/or a further diagnostic measure and wherein said data processor further having implemented an algorithm which allows for recommending cardiac therapy and/or a further diagnostic measure it has been diagnosed that the chest pain in the subject to be tested is caused by cardiac ischemia.

The present invention, finally, encompasses a kit for diagnosing whether chest pain is associated with cardiac ischemia in a subject during or immediately after physical exercise, or not, said kit comprising a detection agent for sFlt1 and a standard which reflect the reference amount as derived from a sample from a reference subject obtained during or immediately after physical exercise, or from a group thereof.

The term "kit" as used herein refers to a collection of the aforementioned components, preferably, provided in separately or within a single container. The container also comprises instructions for carrying out the method of the present invention. These instructions may be in the form of a manual or may be provided by a computer program code which is capable of carrying out the comparisons referred to in the methods of the present invention and to establish a diagnosis accordingly when implemented on a computer or a data processing device. The computer program code may be provided on a data storage medium or device such as a optical storage medium (e.g., a Compact Disc) or directly on a computer or data processing device. Moreover, the kit may, preferably, comprise standards which reflect the reference amounts as described and referred to elsewhere herein in detail. The detection agent is, preferably, immobilized on a carrier, and, preferably, a test stripe.

### Example 1:

The biomarkers sFlt1 and cardiac Troponin T were determined in serum samples obtained from healthy athletes that were subjected to exercise equivalent to the 70%, 90 % and 110 % anaerobic threshold. Blood was taken before exercise, after 30 minutes, after exercise and 1 h, 3 h and 24 h thereafter. At each time point troponin T and sFlT1 were measured, testing was done with diagnostic ELECSYS assays for these markers.

Moreover, the biomarkers were determined in 97 patients with ACS who did not develop Non STEMI (N = 53), or who did develop Non STEMI were tested and who presented within 2 hours after the beginning of chest pain presented to the emergency room.

The following methods and devices were used for determining the amount of the biomarkers referred to herein:

sFlt1 and Troponin T (hsTNT) were determined with sandwich immuno-assays using analyzers from Elecsys. The assays comprise two monoclonal antibodies specific for the respective peptide. The first of these iv biotinylated and the second one in labelled with a Tris(2,2'-bibyridyl)ruthemium (II)-complex. In a first incubation step both antibodies are incubated with the sample. A sandwich complex comprising the peptide to be determined and the two different antibodies is formed. In a next incubation step streptavidin-coated beads are added to this complex. The beads bind the sandwich complexes. The reaction mixture is then aspirated into a measuring cell where the beads are magnetically captured on the surface of the electrode. The application of a voltage then induces a chemilumines-cent emission from the ruthenium complex which is measured by a photomultiplier. The emitted amount of light is dependent on the amount of sandwich complexes on the electrode. sFlt-1 amounts between 10 to 85,000 pg/ml, The high sensitivity Troponin T test used in this study has a sensitivity of 2 pg/ml. The amounts of sFlt1 and Troponin T are given in "pg/ml" herein.

Results are shown in the following Table (Median and 25 and 75 percentile)

| | | | | | | |
|---|---|---|---|---|---|---|
| Timepoint: | 0 | 30 min | after | 1 h | 3h | 24 h |
| Troponin T pg/ml | 3,35 | 3,5 | 4,3 | 5,9 | 14,4 | 4,45 |
| | 2,2 - 5,0 | 2,9 - 5,0 | 3,7 - 6,0 | 4,7 - 7,6 | 8,5 - 14,4 | 3,4-6,4 |
| | | | | | | |
| sFlT1 pg/ml | 68,5 | 116 | 145 | 86,5 | 86,0 | 63,5 |
| | 61 - 77 | 98 - 129 | 117- 156 | 78 - 101 | 79-91 | 50-69 |

As can be seen from the Table, Troponin T increased by more than 400 % which may match with the assumption of Non-STEMI. Peak levels for Troponin T at 3 h were 5,0 pg/ml (4,1 - 7,0) for 70 % anaerobic threshold, 14,4 (8,5 - 22, 8) pg/ml for 90 % anaerobic threshold and 10,6 (6,6 - 19,2) for 110% anaerobic threshold.

sFlT1 as an indicator of ischemia increased also and reached peak levels at the end of exercise, peak levels for sFlT1 were at the end of exercise 92,0 (82 - 101) at 70 % anaerobic threshold, 145 (117 - 156) pg/ml at 90 % anaerobic threshold and 143 (112 - 158) at 110% anaerobic threshold.

The 97 patients with ACS who did not develop Non STEMI (N = 53) and who did develop Non STEMI were tested and who presented within 2 hours after the beginning of chest pain presented to the emergency room for troponin T and sFlT1 as described.

The results are presented in the following Table

### Patients who did not develop Non- STEMI

| | | | | |
|---|---|---|---|---|
| | presentation | 1 - 2 h | 3-4h | 5-24h |
| | | | | |
| troponin T pg/ml | 5,19 | 4,63 | 2,36 | 4,41 |
| | 0,0 - 15,3 | 0,0-17,16 | 0,0 - 10,77 | 0,0 - 19,7 |
| | | | | |
| sFlT1 pg/ml | 288 | 932 | 683 | 180 |
| | 75-3119 | 222 - 2288 | 133-1172 | 106-748 |

### Patients who did develop Non STEMI

| | | | | |
|---|---|---|---|---|
| Troponin T | 21,2 | 120,3 | 112,4 | 431 |
| | 10,5 - 45,6 | 56,8 - 206 | 33 - 203 | 136 - 1207 |
| | | | | |
| sFlT1 | 1566 | 1921 | 905 | 237 |
| | 131 - 5347 | 633 - 5647 | 177 - 1751 | 184 - 476 |

Thus there are increases in sFlT1 and troponin T in healthy athletes as a result of exercise. This needs to be considered if patients with chest pain after exercise present to the emergency room. Therefore, the exercise status has to be taken into account when using sFlt1 as diagnostic marker for cardiac ischemia.

The data suggest that in patients without non-ST elevation myocardial infarction, troponins remain low during the observation period while in Non-STEMI they increase with time. Recent studies however suggest that low troponin T levels can be found in patients with heart failure ( EP 07114174.1/ 1 890 154 an d Omland T. et al NEJM 2009: 361: 1 -10). This makes it difficult to judge if elevated troponin T levels are related to the underlying cardiac disease or related to chest pain or ACS symptoms in case no follow up is available and in follow up of patients after exercise this is even more difficult as troponin T increases might occur that are exercise related but not symptom (chest pain or ACS related). These difficulties are resolved by the determination of sFlT1, sFlT1 levels below approximately 200 pg/ml are related to exercise whereas higher values reflect cardiac ischemia with or without later troponin increase.

### Determination of GDF-15

The biomarker GDF-15 was also determined in serum samples obtained from healthy athletes (see also above). The results are shown in the following table.

| | |
|---|---|
| Before: | 378 (354 - 419) |
| During | 415 (369 - 470) |
| After | 449 (418 - 498) |
| 1 h | 568 (513 - 698) |
| 3h | 539 (473 - 593) |
| 24 h | 379 (339-415) |

Moreover, GDF 15 was determined the above described cohort of ACS patients. The results are shown in the following table.

| | Non MI | MI |
|---|---|---|
| Present. | 820 (632/1321) | 1295 (863/1901) |
| 1-2 h | 839 (598/1281) | 1596 (1070/2498) |
| 3-4h | 805 (606/1253) | 793 (483 / 1196) |
| 5-24h | 852 (647/1228) | 1164 (665/1828) |

In addition to the patients presenting with early ACS 140 patients with STEMI were tested at day one, three und after three months. In contrast to Troponin T which decreased from 1856 (1002/3744) at day one and 1720 (1129, 2714) pg/ml at day 3 to 6.3 ( 3.8, 9.6) at 3 months GDF15 remained fairly stable 1081 (839, 1549) at day 1, 877 (689, 1144) at day 3 and 797 (682, 1117) at three months, thus supporting the data obtained in ACS that GDF 15 remains largely unaffected in early ACS in the early phase of the event (in contrast to troponin T/I or NT-pro BNP).

When the risk criteria of Wollert et al who declares high risk patients to GDF 15 levels above 1800 pg/ml , intermediate risk patients to GDF levels between 1200 - 1800 pg/ml and low risk patients to GDF 15 levels below 1200 pg/ml, the following results apply:

| GDF 15 | total | no MI | MI |
|---|---|---|---|
| | | | |
| Above 1800 | 7 | 6 | 1 |
| | | | |
| 1200-1800 | 10 | 6 | 4 |
| | | | |
| Below 1200 | 80 | 41 | 39 |

The current study focuses on indicators of ischemia and necrosis in subjects who experience chest pain during or after exercise. As shown exercise may induce ischemia which may be recognized by increased sFlT1 levels and thus may result in incorrectly misclassi-fying patients as having myocardial infarction. This can be overcome by using appropriate cur off levels for sFlT1 in patients who experience chest pain during or after sports.

In addition to using appropriate cut off or decision levels for the above markers GDF 15 will be of help to identify individuals with or without evidence of heart failure. A shown GDF 15 is surprisingly not significantly affected by exercise and myocardial infarction and thus gives reliably information on the level of underlying heart failure. Such information is important as an indicator of underlying cardiac disease and also adds in the assessment of the presumed extent of exercise which can not be easily quantitated by taking the medical history. This is further illustrated by the case studies below:

### Case study 1

A 41 year old physically active man takes his round through the woods as he does twice a week. In contrast to his last exercise he notices chest pain after half an hour of running. He decides to visit immediately the emergency room, at presentation his ECG is not abnormal and chest pain had resolved, his sFlT1 is 105 pg/ml, his troponin T is 4 pg/ml and GDF 15 is 520 pg/ml, he is kept in the hospital, 4 hours later his sFlT1 has decreased to 78 pg/ml, his troponin T has increased to 7,8 pg/ml and his ECG remains normal. He is informed that sFlT1and troponin T increases are related to his exercise activities and his chest pain is not of cardiac origin. For safety reasons he is advised to take a cardiac stress test.

### Case study 2

A 52 year old male who had smoked for 15 years but has quitted smoking 8 years ago joins two colleges for exercise, as he did when he was young. After running for 10 minutes chest pain develops which rather intensifies. Therefore, he needs to stop running. He visits the emergency room, his sFlT1 is 320 pg/ml, troponin T is 3.5 pg/ml and GDF 15 is 890 pg/ml. He is suspected of cardiac chest pain, however considered a low risk patient, on follow up troponin T did not increase and sFlT1 decreased to 210 pg/ml. He is released from the hospital without symptoms and recommended to take a cardiac work up because of suspected underlying cardiac disease.

### Case study 3

A 54 year old male develops chest pain shortly after chasing his son, he has multiple risk factors such as smoking and diabetes mellitus and hypertension. Shortly after running he develops chest pain and dyspnoe. He visits the emergency room. His sFlT1 level is 1220 pg/ml, his troponin T level is 9 pg/ml and his GDF15-level is 1950 pg/ml. He is diagnosed which cardiac chest pain and advances heart failure. Thus, the chest pain is due to cardiac ischemia. He receives an angiography and STENT implantation because of one vessel disease with a newly developed plaque which occluded 90 % of the vessel.

### Case study 4

A 54 year old Bavarian male gets excited as he watches a soccer game and one player misses several time the goal and he develops chest pain. He is immediately taken to the emergency room where his ECG is normal, his sFlT1 is 144 pg/ml, troponin T is 6 pg/ml, GDF 15 is 1100 pg/ml, 3 hours later sFlT1 has decreased to 89 pg/ml and troponin T has risen to 21 pg/ml, ECG remains without obvious abnormalities. He is diagnosed with NON-STEMI. The chest pain has been caused by cardiac ischemia, and the subject assigned to the low to intermediate risk group and he is suspected to suffer from cardiac dysfunction. He is advised to stay in the hospital overnight and to have a cardiac evaluation.

### Conclusions

Exercise becomes increasingly popular in developed countries at all ages. One important aspect so far overlooked and also not included into update recommendations is the occurrence of chest pain and acute coronary syndrome associated with exercise (ESC Guidelines, Europ. Heart Journal, see elsewhere herein). The recommendations given in this journal define NonSTEMI as cardiac troponin exceeding the 99th percentile of a normal reference population using an assay with an imprecision of below 10 % at the upper reference limit associated with an undefined fall or rise of troponin. This may be however the case in persons undergoing exercise. Similarly increases of sFlT1 have been documented in patients presenting with ACS with and without the development of ischemia WO/2011/064358). Increases of sFlT1 can however also be observed after exercise. Thanks to the present invention reference amounts could be established for patients exhibiting chest pain and/or ACS during exercise to identify those with true cardiac chest pain. Moreover and in contrast to Kempf et al. (Circulation Research 2006: 98: 351 - 360) significant changes in GDF 15 due to suspected reperfusion injury in early ACS were excluded. Similarly, after exercise GDF 15 is subject to increase, however this increase is not to an extent that it would influence heart failure diagnosis according to the classification of Wollert et al (Circulation 2007: 115: 962 - 971) who took samples more than 12 hours after the cardiac event. Thus GDF 15 concentrations can be used in patients who experience chest pain and or ACS to dissect them into low, intermediate and high risk patients and to build individualized intervention strategies.

## Claims

1. A method for diagnosing whether chest pain is associated with cardiac ischemia in a subject during or immediately after physical exercise, comprising
a) determining the amount of soluble fms-like tyrosine kinase-1 (sFlt1) in a sample obtained during or immediately after physical exercise from a subject exhibiting chest pain,
wherein the reference amount for diagnosing whether chest pain is associated with cardiac ischemia is derived from a sample from a reference subject obtained during or immediately after physical exercise, or from a group thereof.

2. The method of claim 1, further comprising the step b) of comparing the, thus, determined amount of sFlt1 to a reference amount, whereby it is diagnosed whether chest pain is associated with cardiac ischemia.

3. The method of claim 1 and 2, wherein the sample obtained immediately after exercise has been obtained not more than 90 minutes after physical exercise, in particular not more than 45 minutes after physical exercise.

4. The method of any one of claims 1 to 3, wherein said physical exercise is endurance exercise.

5. The method of any one of claims 1 to 4, wherein the exercise intensity is at least 70% of the anaerobic threshold, in particular at least 90%, or at least 110% of the anaerobic threshold.

6. The method of any one of claims 1 to 5, wherein the subject participated in physical exercise for at least 30 minutes.

7. The method of any one of claim 1 to 6, wherein the reference subject is known to exhibit chest pain not associated with cardiac ischemia.

8. The method of claim 7, wherein an amount of sFlt1 in a sample of the subject which is essentially the same or which is decreased as compared to the reference amount, indicates that the subject exhibits chest pain not associated with cardiac ischemia.

9. The method of any one of claims 1 to 8, wherein the sample is blood, serum or plasma.

10. The method of any one of claims 1 to 9, further comprising the determination of GDF-15 in the sample from the subject, and comparing, the, thus, determined amount to a reference amount.

11. Use of sFlt1, or of a detection agent, which specifically binds thereto, in a sample obtained during or immediately after physical exercise from a subject exhibiting chest pain, for assessing whether the chest pain is associated with cardiac ischemia.

12. A device for diagnosing whether chest pain is associated with cardiac ischemia in a subject during or immediately after physical exercise, or not, said device comprising:
a) an analyzing unit comprising a detection agent for sFlt1 which allows for the determination of the amount of sFlt1; and
b) an evaluation unit comprising a data processor having implemented an algorithm for comparing the amounts determined by the analyzing unit with reference amounts stored in a database in order to establish a diagnosis indicating whether chest pain is associated with cardiac ischemia in a subject during or immediately after physical exercise, or not, wherein the reference amount is derived from a sample from a reference subject obtained during or immediately after physical exercise, or from a group thereof

13. A kit for diagnosing whether chest pain is associated with cardiac ischemia in a subject during or immediately after physical exercise, or not, said kit comprising a detection agent for sFltl and a standard which reflect the reference amount as derived from a sample from a reference subject obtained during or immediately after physical exercise, or from a group thereof.

## Amended claims

### Amended claims in accordance with Rule 137(2) EPC.

**1.** A method for diagnosing whether chest pain is associated with cardiac ischemia in a subject during or immediately after physical exercise, comprising
a) determining the amount of soluble fms-like tyrosine kinase-1 (sFlt1) in a sample obtained during or immediately after physical exercise from a subject exhibiting chest pain,
wherein the reference amount for diagnosing whether chest pain is associated with cardiac ischemia is derived from a sample from a reference subject obtained during or immediately after physical exercise, or from a group thereof.

**2.** The method of claim 1, further comprising the step b) of comparing the, thus, determined amount of sFlt1 to a reference amount, whereby it is diagnosed whether chest pain is associated with cardiac ischemia.

**3.** The method of claim 1 and 2, wherein the sample obtained immediately after exercise has been obtained not more than 90 minutes after physical exercise, in particular not more than 45 minutes after physical exercise.

**4.** The method of any one of claims 1 to 3, wherein said physical exercise is endurance exercise.

**5.** The method of any one of claims 1 to 4, wherein the exercise intensity is at least 70% of the anaerobic threshold, in particular at least 90%, or at least 110% of the anaerobic threshold.

**6.** The method of any one of claims 1 to 5, wherein the subject participated in physical exercise for at least 30 minutes.

**7.** The method of any one of claim 1 to 6, wherein the reference subject is known to exhibit chest pain not associated with cardiac ischemia.

**8.** The method of claim 7, wherein an amount of sFlt1 in a sample of the subject which is essentially the same or which is decreased as compared to the reference amount, indicates that the subject exhibits chest pain not associated with cardiac ischemia.

**9.** The method of any one of claims 1 to 8, wherein the sample is blood, serum or plasma.

**10.** The method of any one of claims 1 to 9, further comprising the determination of GDF-15 in the sample from the subject, and comparing, the, thus, determined amount to a reference amount.

**11.** Use of sFlt1, or of a detection agent, which specifically binds thereto, in a sample obtained during or immediately after physical exercise from a subject exhibiting chest pain, for assessing whether the chest pain is associated with cardiac ischemia.

**12.** A device for diagnosing whether chest pain is associated with cardiac ischemia in a subject during or immediately after physical exercise, or not, said device comprising:
a) an analyzing unit comprising a detection agent for sFlt1 which allows for the determination of the amount of sFlt1; and
b) an evaluation unit comprising a data processor having implemented an algorithm for comparing the amounts determined by the analyzing unit with reference amounts stored in a database in order to establish a diagnosis indicating whether chest pain is associated with cardiac ischemia in a subject during or immediately after physical exercise, or not, wherein the reference amount is derived from a sample from a reference subject obtained during or immediately after physical exercise, or from a group thereof.
